(19) Europäisches Patentamt
European Patent Office
Office européen des brevets

(11) Publication number : **0 576 287 A1**

# (12) EUROPEAN PATENT APPLICATION

(21) Application number : **93304962.9**

(22) Date of filing : **24.06.93**

(51) Int. Cl.$^5$ : **A61K 7/04,** A61K 7/06, A61K 7/48

(30) Priority : **25.06.92 GB 9213472**

(43) Date of publication of application : **29.12.93 Bulletin 93/52**

(84) Designated Contracting States : **AT BE CH DE DK ES FR GB GR IE IT LI NL PT SE**

(71) Applicant : **UNILEVER PLC**
**Unilever House Blackfriars**
**London EC4P 4BQ (GB)**
(84) **GB IE**

(71) Applicant : **UNILEVER N.V.**
**Weena 455**
**NL-3013 AL Rotterdam (NL)**
(84) **BE CH DE DK ES FR GB GR IE IT LI NL PT SE AT**

(72) Inventor : **Hagan, Desmond Bernard**
**35 Hookstone Drive, Little Sutton**
**South Wirral, Cheshire L66 4TE (GB)**
Inventor : **Taylor, Anthony Philip**
**Lindendreef 10**
**NL-3137 CM Vlaardingen (NL)**
Inventor : **Joiner, Andrew**
**36 St Michael's Road**
**Liverpool L17 7AR (GB)**

(74) Representative : **Ford, Michael Frederick et al**
**MEWBURN ELLIS 2 Cursitor Street**
**London EC4A 1BQ (GB)**

## (54) Cosmetic compositions.

(57) A composition for application to human skin to enhance elasticity contains as active ingredient a sulphur-containing compound of the general formula :

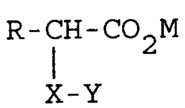

in which M is hydrogen or a water-solubilising cation, X is S,

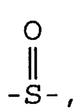

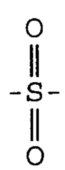

or

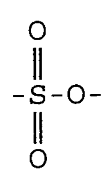

and Y is alkyl or alkenyl of up to 4 carbon atoms, or else -XY is -SH.

EP 0 576 287 A1

This invention relates to cosmetic compositions for topical application to skin, hair and nails, especially to skin.

A soft, supple and flexible skin has a marked cosmetic appeal and is an attribute of normal functioning epidermis. The outer layer of the epidermis, i.e. the stratum corneum, can, however, become dry and flaky following exposure to adverse climatic conditions, or excessive contact with detergents or solvents which result in loss of skin moisture, so that the skin loses its soft, supple and flexible characteristics. Emollients such as fats, phospholipids and sterols have in the past been used to soften dry skin, but it is apparent that these emollients are only partially effective as a remedy for this type of condition. Also, topical application to the skin of classical humectants is unlikely to alleviate this problem since they are not particularly skin substantive and are generally rinsed from the skin during washing.

It has been proposed in US 4 105 782 (Yu and Van Scott) to use amides or ammonium salts of α-hydroxyacids in the treatment of acne or dandruff and, in the Yu & Van Scott patents, US 4 105 783 and US 4 197 316, to use such compounds in the treatment of dry skin. US 4 234 599 (Yu & Van Scott) discloses the use of α-hydroxyacids, and their esters or amine salts in the treatment of keratoses.

In US 4 363 815 (Yu & Van Scott) it is proposed to use α-hydroxyacids or β-hydroxyacids or ketoacids or their derivatives, including inorganic salts, in a composition for treating skin conditions. The acids referred to include some short chain α-hydroxyacids with sulphur-containing substituents at beta and gamma positions.

GB 1471679 (Avon) discloses the use of alkali metal salts of $C_2$ -$C_5$ α-hydroxy carboxylic acids in moisturising compositions.

EP-B-7785 (Unilever) discloses the use of certain longer chain 2-hydroxyalkanoic acids as active agents included in compositions for topical application to skin. These give benefits which include increased elasticity of the skin, particularly the stratum corneum. EP-A-442708 (Unilever) discloses unsaturated analogues of these compounds for the same purpose.

We have now found that certain other 2-substituted monocarboxylic acids, and their salts, are also able to give a beneficial increase in the elasticity of skin.

We have also found that these compounds display a useful antimicrobial activity.

According to a first aspect of the present invention there is provided a cosmetically acceptable composition suitable for topical application to human skin, hair or nails which comprises:

(i) from 0.1 to 99.9% by weight of an acid or salt thereof having the general formula

$$\begin{array}{l} R-CH-CO_2M \\ \quad\ \ | \\ \quad X-Y \end{array} \qquad (I)$$

in which

R is a substituted or unsubstituted alkyl or alkenyl chain of 4 to 28 carbon atoms, optionally interrupted by a heteroatom;

M is hydrogen or a water-solubilising cation;

X is -S-,

$$\begin{array}{c} O \\ \| \\ -S- \end{array}, \quad \begin{array}{c} O \\ \| \\ -S- \\ \| \\ O \end{array} \quad or \quad \begin{array}{c} O \\ \| \\ -S-O- \\ \| \\ O \end{array}$$

and Y is an alkyl or alkenyl group of up to 4 carbon atoms or, if X is -S-, Y may additionally be hydrogen,

(ii) from 0.1 to 99.9% of a cosmetically acceptable vehicle.

The group R may be branched or (preferably) linear. It preferably has from 4 to 16 carbon atoms, preferably at least 6 carbon atoms, and more preferably it has 6 or 8 carbon atoms.

The group X is preferably sulphur without attached oxygen, i.e. -S- and Y is then preferably hydrogen or lower alkyl of 1 to 4 carbon atoms.

In a further aspect, the invention provides a method of treating skin by applying thereto a compound of formula (I) especially as a cosmetic composition as specified above. In yet another aspect the invention provides the use of a compound of formula (I) above for the preparation of a composition for application to human skin.

Preparative Routes

Compounds of the general formula (I) in which -XY is a thiol group -SH can be prepared from the corresponding 2-bromo substituted acid by reaction with sodium thiosulphate to yield a Bunte salt. This is then hydrodysed under aqueous conditions. Alternatively it can be hydrodysed in alcohol, e.g. ethanol, to yield an ester which is then saponified. The reaction scheme is:

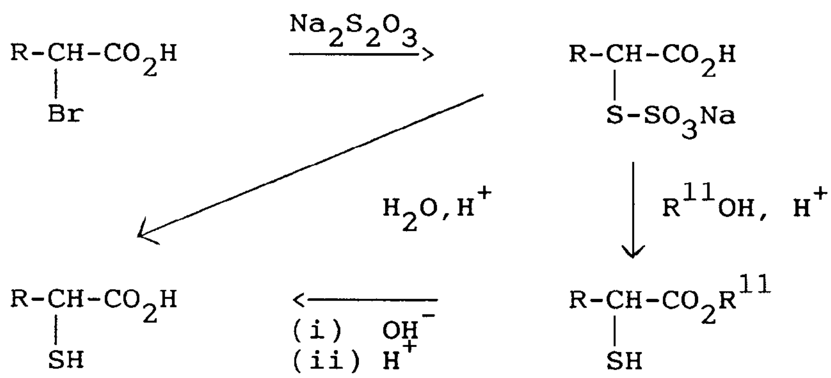


Compounds of the general formula (I) in which -XY is an alkyl thio group can be prepared from the corresponding acids without 2-substitution. The acid is reacted with two equivalents of lithium diisopropylamide (LDA) to yield a dianion. This is then quenched with the appropriate alkyldisulphide:

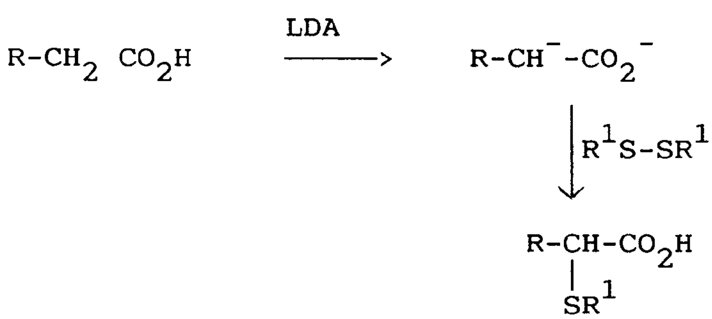


The above alkyl thio compounds can be oxidised to compounds in which X is a sulphoxide group. The route consists of protective esterification, oxidation for which metaperiodate is suitable, and hydrolystic removal of the ester to the acid:

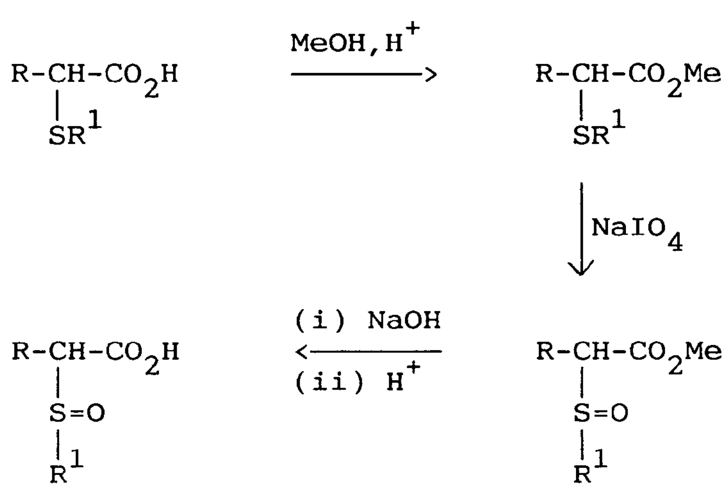

Further oxidation may provide a route onwards to the corresponding sulphones. Such compounds in which Y is methyl can alternatively be prepared by alkylation of ethylmethane sulphonyl acetate with an alkyl halide, followed by hydrolysis of the resulting ester.

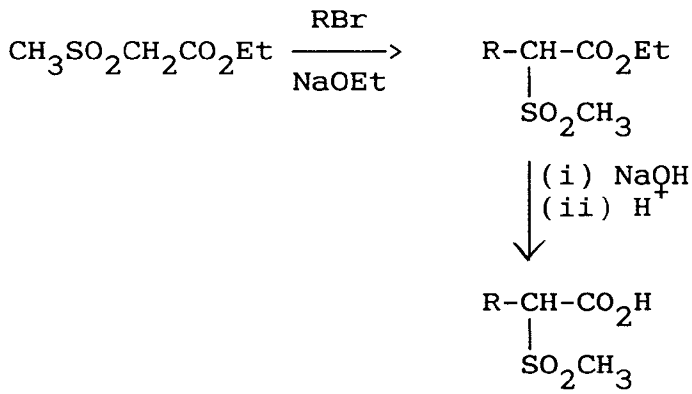


Another route to compounds in which X is $-SO_2-$ commences with a 2-halocarboxylic acid. This is first subjected to protective esterification after which it is reacted with an alkylsulphonyl hydrazide in a procedure derived from that published by Ballini et al, Tetrahedron, (1989), P6791. The product is then separated from a by-product and hydrolysed. The overall scheme is:

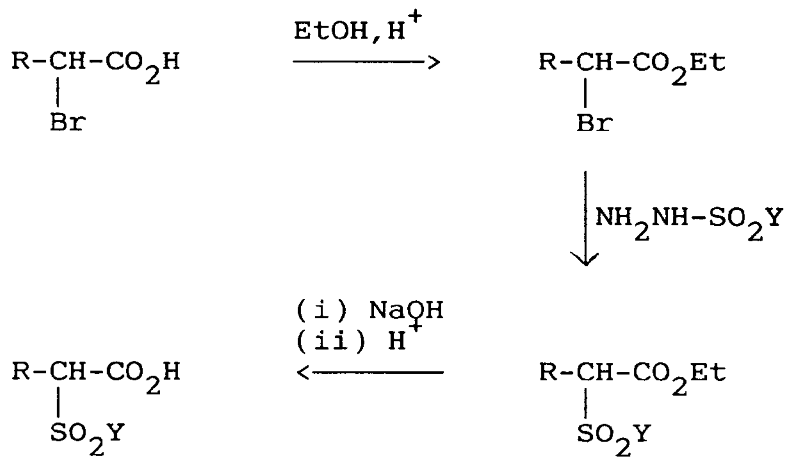


Compounds of the formula (I) in which R includes olefinic unsaturation and X is methylthio may be prepared starting with ethyl methylthio acetate. This is oxidised to ethyl methanesulphinylacetate, followed by a thio-ene reaction with an olefin, and hydrolysis of the product. (This is based on a reaction disclosed by Tamara et al, Tetrahedron Letters, (1981), P 81). The reaction scheme is

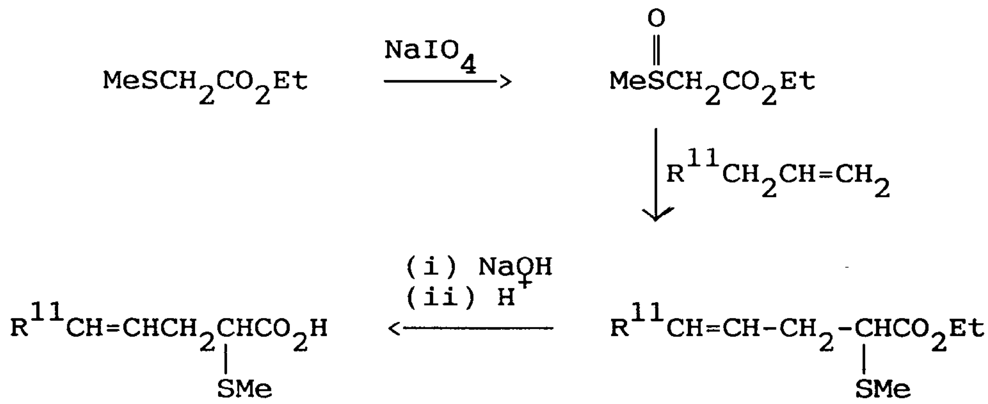


Compositions according to this invention preferably contain a compound of formula (I) in an amount from 0.1 to 90%, more preferably from 0.5 to 20%. The upper boundary on the amount may be less, e.g. 15%, 10% or even 5%. Particularly, preferred is from 1 to 5% by weight of the composition.

<u>The Cosmetically Acceptable Vehicle</u>

The composition according to the invention also comprises a cosmetically acceptable vehicle, the selection of which will depend on the required product form of the composition. Typically, the vehicle will be chosen from diluents, dispersants or carriers for the 2-substituted acid of formula (I), so as to ensure an even distribution of it when applied to the skin.

Compositions according to this invention can include water as a vehicle, usually with at least one other cosmetically-acceptable vehicle.

Vehicles other than water that can be used in compositions according to the invention can include various liquids or solids which perform a function such as emollients, solvents, humectants, thickeners and powders. Examples of each of these types of vehicles, which can be used singly or as mixtures of one or more vehicles, are as follows:

Emollients, such as stearyl alcohol, glyceryl monoricinoleate, glyceryl monostearate, propane-1,2-diol, butane-1,3-diol, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernel oil, rapeseed oil, safflower seed oil, soybean oil, sunflower seed oil, olive oil, sesame seed oil, coconut oil, arachis oil, castor oil, acetylated lanolin alcohols, petroleum, mineral oil, butyl myristate, isostearic acid, palmitate acid, isopropyl linoleate, lauryl lactate, myristyl lactate, decyl oleate, myristyl myristate; silicone oil, notably volatile polydimethylsiloxane accompanied by a silicone surfactant to form a water-in-silicone oil emulsion;

Propellants, such as trichlorofluoromethane, dichlorodifluoromethane, dichlorotetrafluoroethane, monochlorodifluromethane, trichlorotrifluoroethane, propane, butane, isobutane, dimethyl ether, carbon dioxide, nitrous oxide;

Solvents, such as ethyl alcohol, methylene chloride, isopropanol, acetone, castor oil, ethylene glycol monoethyl ether, diethylene glycol monobutyl ether, diethylene glycol monoethyl ether, dimethyl sulphoxide, dimethyl formamide, tetrahydrofuran;

Humectants, such as glycerin, sorbitol, sodium 2-pyrrolidone-5-carboxylate, soluble collagen, dibutyl phthalate, gelatin;

Powders, such as chalk, talc, fullers earth, kaolin, starch, gums, colloidal silicon dioxide, sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The cosmetically acceptable vehicle will usually form from 10 to 99.9%, preferably from 50 to 99% by weight of the composition, and can, in the absence of other cosmetic adjuncts, form the balance of the composition.

The vehicle should have a pH such that the compound of formula (I) is at least partly, better mostly, in free acid form rather than salt form.

## Cosmetic Adjuncts

Examples of other conventional adjuncts, some of which can also function as vehicles, that may optionally be employed, include non-volatile silicones; silicone polymers; preservations, such as para-hydroxy benzoate esters; humectants, such as butane-1,3 diol, other alkane diols, glycerol, sorbitol, polyethylene glycol; stabilisers, such as sodium chloride or ammonium chloride; buffer system, such as lactic acid together with a base such as sodium hydroxide; oils and waxes, such as avocado oil, evening primrose oil, sunflower oil, beeswax, ozokerite wax, paraffin wax, lanolin, lanolin alcohol; emollients; thickeners; activity enhancers; colourants; whiteners; perfumes; emulsifiers; sunscreens; bactericides and water.

Cosmetic adjuncts can form up to 50% by weight of the composition and can conveniently form the balance of the composition.

## Process for preparing the composition

The invention also provides a process for the preparation of a composition for topical application to human skin which comprises the step of incorporating the 2-substituted acid of formula (I) above, into the composition, together with a cosmetically acceptable vehicle.

## Use of the composition

The composition according to the invention is intended primarily as a product for topical application to human skin, particularly dry skin, when repeated application can alleviate the dry condition, and restore the skin to a more natural, soft, supple, healthy state. The composition can also be used to treat the hair, including the scalp, and finger and toe nails.

In use, a small quantity of the composition, for example from 1 to 5 ml, is applied to the affected area of skin, hair or nails, from a suitable container or applicator and, if necessary, it is then spread over and/or rubbed into the skin, hair or nails using the hand or fingers or a suitable device.

## Product forms and packaging

The topical skin treatment composition of the invention can be formulated as a fluid, for example in a product such as a lotion, with or without an applicator such as a roll-ball applicator, or a propellant-driven aerosol device or a container fitted with a pump to dispense the composition, for example as a mousse or simply for storage in a non-deformable bottle or squeeze container. Alternatively, the composition of the invention may be solid for example, as a bar or tablet, such as a soap bar, or semi-solid, for example as a cream, lotion, gel or ointment, for use in conjunction with a suitable applicator, or simply for storage in a tube or lidded jar.

The invention accordingly also provides a closed container containing a cosmetically acceptable composition as herein defined.

## EXAMPLES

In the examples below all parts and percentages are by weight unless otherwise stated.

Proton n.m.r. spectra were recorded at 360mHz in deuterochloroform as solvent, except in the case of the Bunte salt where deuteromethanol was used.

## EXAMPLE 1

### Preparation of 2-methylthiooctanoic acid

Octanoic acid (5.04g, 0.035 mol) was dissolved in dry tetrahydrofuran (125ml) and cooled to 0-5°C (ice/water bath) with stirring under a nitrogen atmosphere. Lithium di-isopropylamide (1.99M, ex Lithco, 37ml, 0.074 mol) was syringed into the mixture and stirred at 0-5°C for 30 minutes. The mixture was then stirred at 40-50° for 1.5 hours to give a clear orange solution. After cooling to room temperature, methyl disulphide (19.7g, 0.21 mol) was added dropwise and then left to stir at room temperature overnight. The mixture was evaporated to dryness and water (50 ml) added. This was acidified to pH 1 (conc HC1) and extracted with ethyl acetate (3 x 100 ml). The combined organic extracts were washed with water (100 ml) and brine, dried ($MgSO_4$) and evaporated to give an orange oil. This was distilled to give the title compound (3.84g, 58%) as a pale yellow liquid, b.p. = 165-185°C/1.2 mmHg.

Infra-red spectrum showed peaks at (liq. film), 3000 (br), 2930, 2860, 1700, 1410, 1280, 1190, 1110 and 930 (br) $cm^{-1}$;
Proton n.m.r. showed δ:0.90 (3H, t, J=6.5Hz, $C\underline{H}_3CH_2$) 1.30-1.50 (8H, m, $CH_2$), 1.70 (1H, m, $C\underline{H}_2CHS$) 1.90 (1H, m $C\underline{H}_2CHS$) 2.20 (3H, s, $CH_3S$), 3.20 (1H, t, J=7.5Hz, $C\underline{H}S$).
Mass spectrum showed the preducted molecular ion.

EXAMPLE 2

Preparation of 2-methylthiodecanoic acid

Decanoic acid (6.0g, 0.035 mol) was dissolved in dry tetrahydrofuran (125ml) and cooled to 0°C with stirring under a nitrogen atmosphere. Lithium diisopropylamide (1.99M, 37ml, 0.074 mol) was syringed into the reaction mixture and stirred at 0°C for 30 min. The mixture was then warmed to 50°C and stirred at that temperature for 90 min. After cooling to ambient temperature, methyl disulphide (10.7g, 0.21 mol) was added and the resulting mixture stirred overnight (17h). The solvent was removed under reduced pressure and the residue diluted with water (50ml) and acidified to pH 1 with concentrated hydrochloric acid. The aqueous solution was extracted with ethyl acetate (3 x 100ml), the organic layers combined, washed with water, then brine, dried ($MgSO_4$) and evaporated to give an orange oil. The oil was distilled to give the title compound (4.90g, 64%) as a yellow oil.

Proton n.m.r. showed δ:0.90 (3H, t, $CH_3$); 1.30-1.50 (8H, m, $(CH_2)_4$; 1.70 (1H, m, $CH_2CHS$); 1.90 (1H, m, $CH_2CHS$); 2.20 (3H, s, $CH_3S$); 3.20 (1H, t, CHS);
IR (liquid film) showed peaks at 3100(br), 2930, 2860, 2650(br), 1700, 1460, 1415, 1380, 1280, 1190, 1115, 960. 930, >25 & $685cm^{-1}$.

EXAMPLE 3

Octanoic acid 2-Bunte Salt

2-Bromo-octanoic acid (10 g, 0.045 mol) in ethanol (25 ml) was treated with $Na_2S_2O_3.5H_2O$ (12.41 g, 0.050 mol) in water (45 ml). The mixture was heated under reflux for 1.5 hours, cooled and evaporated to dryness to give a white crystalline solid. Ethanol (200 ml) was added and the mixture boiled to dissolve organics, filtered hot, cooled and evaporated. The crude material was recrystallised from EtOH to give the product (4.09 g, 37%) as white crystals, m.p. 222-232°C (dec).

Infra-red spectrum showed peaks at (nujol) 2750, 1655, 1410, 1355, 1330, 1210, 1195, 1160, 1125, 995 and 600 $cm^{-1}$.
Proton n.m.r. showed δ:0.90 (3H, t, J=6.5Hz, $CH_3$), 1.25-1.45 (8H, m, $CH_2$) 1.95 (2H, m, $C\underline{H}_2$ CHS), 3.95 (1H, t, J=7.2Hz, CHS).
Mass spectrum showed the predicted molecular ion.

Ethyl 2-thiol octanoate

The above Bunte salt (2.97 g, 0.011 mol) was heated under reflux with ethanol (350 ml) and concentrated hydrochloric acid (25 ml) for 1 hour. Cooled and the mixture was poured into water (800 ml) when the solid originally present ($NaHSO_4$) went into solution and a milky liquid resulted. This was extracted with ethyl acetate (4 x 250 ml). The combined organic extracts were washed with water (200 ml) and brine, dried ($MgSO_4$) and evaporated. This was distilled under reduced pressure to give the product (1.59 g, 71%) as a colourless liquid, bp 130-150°C/1.1mm Hg.

Infra-red spectrum showed peaks at (liq. film) 3200(br), 2960, 2920, 2860, 2560 (S-H), 1735 (ester C=O), 1460, 1370, 1335, 1160, 1030, 860 and 725 $cm^{-1}$.
Proton n.m.r. showed, δ:0.90 (3H, t, J=7.0Hz, $C\underline{H}_3CH_2CH_2$), 1.29 (3H, t, J=7Hz, $C\underline{H}_3CH_2O$) 1.25-1.45 (8H, m, $CH_2CH_2$), 1.75 (1H, m, $C\underline{H}_2CHSH$), 1.90 (1H, m, $C\underline{H}_2CHSH$), 2,03 (1H, d, J=9Hz, SH), 3.30 (1H, m, $C\underline{H}SH$) 4.20 (2H, q, J=9.5Hz, $CH_3C\underline{H}_2O$).
Mass spectrum showed the predicted molecular ion.

2-Thiol Octanoic Acid

Ethyl 2-thiol octanoate, as above (2.40g, 0.01 mol) was heated under reflux with NaOH (3.6g, 0.09 mol)

water (12.5 ml) and methanol (12.5 ml) for one hour under a nitrogen atmosphere. The mixture was cooled and diluted with water (50 ml) to obtain a clear solution. This was extracted with hexane (50 ml) and the aqueous layer was acidified to pH 1 with 6N HCl. The mixture was extracted with ethyl acetate (3 x 75 ml). The combined ethyl acetate extracts were washed with water (50 ml), brine, dried ($MgSO_4$) and evaporated to give the product (1.56 g, 89%) as a colourless oil.

Infra red spectrum showed peaks at liq. film) 2920, 2660 (S-H) 1705, 1460, 1415, 1375, 1280 and 925 $cm^{-1}$.

Proton n.m.r. showed δ:0.90 (3H, t, C<u>H</u>$_3$CH$_2$), 1.75 (1H, m, <u>CH</u>$_2$CHS), 1.90 (1H, m, C<u>H</u>$_2$CHS), 1.30 (8H, m, 4x$CH_2$), 3.30 (1H, m, CHS).

EXAMPLE 4

Alternative preparation of 2-Thiol octanoic acid

The Bunte salt, prepared as in the previous example, (5.0 g, 0.018 mol) was heated under reflux with water (500 ml) and concentrated hydrochloric acid (50ml) for 1 hour. The mixture was cooled and extracted with ethyl acetate (3 x 150 ml). The combined organic extracts were washed with water, brine, dried ($MgSO_4$) and evaporated to give the product (1.20 g, 40%) as a colourless oil which was characterised as in the previous Example.

EXAMPLE 5

Methyl-2-methylthio octanoate

2-methylthio octanoate acid prepared as in Example 1 (3.00g, 0.015% mol) together with concentrated sulphuric acid (2ml) in methanol (50 ml) were heated at reflux temperature (80°C) for 5 hours and then left to stir at ambient temperature overnight. In the morning the methanol was removed by evaporation under reduced pressure to leave an oily residue. The residue was dissolved in ethyl acetate (100 ml) and then washed with saturated sodium bicarbonate (50 ml) and brine (50 ml). The organic layer was separated, dried over magnesium sulphate and concentrated in vacuo to give the title compound (2.84g, 88%) as an amber coloured liquid.

Proton n.m.r. showed δ:0.88 (3H, t, $CH_3$; 1.24 (8H, bs, $(CH_2)_4$); 1.68 (1H, m, CH); 1.88 (1H, m, CH); 2.12 (3H, s, $CH_3S$); 3.19 (1H, d, CHS); 3.75 (3H, s, $CO_2CH_3$).

Infra red spectrum (liquid film) showed peaks at 2900, 2850, 1735, 1440, 1270, 1160 $cm^{-1}$.

Methyl-2-methanesulphinyloctanoate

Methyl-2-methylthio octanoate, (2.20g, 0.0108ml) was added dropwise with stirring to a freshly prepared ice cooled solution of sodium metaperiodate (2.42g, 0.0113 mol) in 1:1 methanol/water (50 ml). The reaction mixture was left to warm up to ambient temperature and stirred overnight (24h). The inorganic materials which precipitated during the reaction were discarded and the methanol solvent was removed by evaporation. The remaining aqueous residue was extracted into dichloromethane (100ml) and then washed with saturated brine solution (50ml). The organic layer was collected, dried over magnesium sulphate and concentrated under reduced pressure to give a 1:1 diastereomeric mixture of the title compound. The yield was 2.37g, 89%.

Proton n.m.r. showed δ:0.99 (3H, t, $CH_3$); 1.32 (8H, bm, $(CH_2)_4$); 1.94 (2H, m, $CH_2$); 2.63*, 2.59* (3H, s, $SOCH_3$); 3.50*, 3.56* (1H, d, CHSO); 3.78*, 3.80* (3H, s, $CO_2CH_3$). Asterisks indicate twin values associated with the presence of diastereomers.

2-Methanesulphinyloctanoic acid

Methyl-2-methanesulphinyloctanoate, (1.59g, 0.00723 mol) in methanol (4 ml) was added dropwise to an aqueous methanolic solution of sodium hydroxide (1.74g NaOH, methanol 4 ml, water 4 ml). The cloudy suspension was stirred at room temperature for 24 hours and then acidified to pH 1 with concentrated hydrochloric acid. An additional 5 ml of water was added to make the solution more mobile and then it was extracted with ethyl acetate (2x30 ml). The organic layers were combined, dried over magnesium sulphate and concentrated in vacuo to leave the title compound (1.07g, 72%) as an amber oil.

Proton n.m.r. showed δ:0.99 (3H, t, $CH_3$); 1.20-1.65 (8H, bm, $(CH_2)_4$); 1.90 (1H,, m, CH); 2.10 (1H, m, CH); 2.62*, 2.80* (3H, s, $SOCH_3$); 3.34*, 3.75 (1H, dd, $CH_3SO$); 8.06 (1H, bs, OH).

Infra red spectrum (Nujol mull) showed peaks at 2940, 2860, 1700, 1460, 1380, 1280, 1240, 980, 950 $cm^{-1}$.

EXAMPLE 6

Ethyl-2-methanesulphonyloctanoate

Commercial ethyl-2-methanesulphonylacetate (3.66g, 0.022 mol) was added to a freshly prepared solution of sodium ethoxide (0.64 g sodium in 20 ml of ethanol) at ambient temperature. The mixture was taken to reflux (80°C). 1-Bromohexane (4.73g, 0.0331 mol) in ethanol (10 ml) was then added dropwise and the mixture heated at reflux temperature for 5 hours. The progress of the reaction was monitored by TLC (40% EtOAc/hexane). After stirring at room temperature for a further 12 hours, an additional 0.2 equivalents of sodium ethoxide was added and the reaction mixture heated at reflux for 4 hours. The precipitated sodium bromide was then filtered and the ethanolic filtrate concentrated under reduced pressure to leave a yellow residue. The residue was dissolved in ethyl acetate (100 ml) washed with brine (2 x 50 ml) dried over $MgSO_4$ and concentrated in vacuo to the crude product as a yellow liquid. The crude product was purified by silica-gel chromatography to leave the title compound (3.26g, 60% as a colourless liquid.

Proton n.m.r. showed δ:0.88 (3H, t, $CH_3$); 1.26 (8H, bm, $(CH_2)_4$); 3.02 (3H, $CH_3SO_2$); 3.74 (1H, dd, CH); 4.30 (2H, q, $OCH_2$).

2-Methanesulphonyloctanoic acid

Ethyl-2-methanesulphonyloctanoate, as above, (2.46g, 0.00984 mol) was heated under reflux in 20% w/v aqueous methanolic sodium hydroxide (4.08g, NaOH, 10ml water, 10 ml methanol) for 3.5 hours. The solution was cooled to ambient temperature, diluted with water (15ml) and then acidified to pH 1 with concentrated hydrochloric acid. The resulting cloudy solution was then extracted with ethyl acetate (2 x 50 ml). The organic layers were combined, dried over magnesium sulphate and evaporated under reduced pressure to give 2.03g (93%) of the title compound as a white solid.

Proton n.m.r. showed δ:0.88 (3H, t, $CH_3$); 1.20-1.54 (8H, bm, $(CH_2)_4$); 2.12 (2H, bm, $CH_2$); 3.08, (3H, s, $CH_3SO_2$); 3.82 (1H, dd, CH); 9.62 (1H, bs, OH).

EXAMPLE 7

Ethyl-2-methanesulphonylhexanoate

Commercial ethyl-2-methanesulphonylacetate (5.0g, 0.03 mol) was added to a freshly prepared solution of sodium ethoxide (2.04g, 0.015 mol). The resulting clear solution was then heated at refluxed for 20 min during which time 1-bromobutane (4.5g, 0.033 mol) in 5 ml of ethanol was added dropwise. The progress of the reaction was monitored by TLC (40% EtOAc/hexane, visualise with $I_2$). After 5 hours sodium ethoxide (0.0133 mol) was added and the reaction mixture was left to stir at ambient temperature for a further 12 hours. The reaction mixture was then diluted with ethanol (100 ml) and any sodium bromide precipitate removed. The resulting cloudy yellow suspension was concentrated under reduced pressure to leave an orange residue. The residue was dissolved in ethyl acetate (100 ml), washed with brine (2 x 50 ml), dried over $MgSO_4$ and concentrated in vacuo to leave the title compound as an orange liquid (5.27g). The crude product was purified by silica-gel chromatography. Yield (4.83g) 73%.

Proton n.m.r. showed δ:0.92 (3H, bt, $CH_3$); 1.34 (3H, t, $CH_3$); 1.36 (4H, m, $(CH_2)_4$); 3.02 (3H, s, $CH_3SO_2$); 3.74 (1H, dd, CH); 4.31 (2H, q, $OCH_2$).

2-Methanesulphonylhexanoic acid

Ethyl-2-methanesulphonylhexanoate, as above, (1. 83g, 0.00825 mol) was heated under reflux in a 20% w/v solution of aqueous methanolic sodium hydroxide (3.5g of NaOH in 8 ml methanol, 10 ml water) for 2 hours. The solution was then cooled to room temperature, diluted with water (12 ml) and acidified to pH 1 with conc. hydrochloric acid. The cloudy solution which resulted was extracted with tehyl acetate (2 x 50ml). The organic phases were combined, dried over sodium sulphate and evaporated under reduced pressure to give the title compound as an orange oil, 1.31 g, 82%.

Proton n.m.r. showed δ:0.94 (3H, bt, $CH_3$); 1.42 (4H, bm, $(CH_2)_2$); 2.12 (2H, bm, $CH_2$); 3.08 (3H, s, $CH_3SO_2$); 3.87 (1H, dd, CH); 8.90 (1H, bs, OH).

EXAMPLE 8

Ethyl-2-bromooctanoate

To a 250 ml flask fitted with a condenser and drying tube, 2-bromooctanoic acid (18.2g, 0.0816 mol), ethanol (180 ml) and concentrated sulphuric acid (10 ml) were added. The resulting solution was heated at reflux temperature for 4.5 hours. The ethanol was then removed in vacuo to leave a cloudy liquid. The crude product was dissolved in diethyl ether (200 ml), washed with saturated sodium bicarbonate solution (100ml) and brine (100 ml). The organic phase was dried over magnesium sulphate and concentrated under reduced pressure to give the title compound (7) as a pale yellow liquid (16.4g 80%).

Proton n.m.r. showed δ:0.88 (3H, t, $CH_3$); 1.22-1.50 (11H, bs, $CH_2)_4$, $CH_3CH_2O$); 2.04 (2H, m, $CH_2CHBr$); 4.22 (3H, m, CHBr, $COCH_3$).

Infra red spectrum (Liquid film) showed peaks at 2970, 2860, 1740, 1465, 1370, 1260, 1150, 1030 $cm^{-1}$.

Ethyl-2-methylsulphonyloctanoate

Commercial methanesulphonylhydrazide (1.0g, 0.0091 mol), ethyl-2-bromooctanoate, prepared as above, (4.5g, 0.0182 mol, 2Eq) and sodium acetate 4.51 g, 0.055 mol) in ethanol (50 ml) were added to a 250ml flask fitted with a condener and drying tube. The resulting mixture was heated at reflux temperature for 5 hours and then cooled to ambient temperature and left to stir over the weekend (17 hours). The ethanol was then removed by evaporation to leave an oily residue which was diluted with ethyl acetate (50 ml) and washed with brine (50 ml). The organic layer was collected, dried and evaporated to leave a yellow oily/liquid which contained the title compound together with ethyl octanoate by-product and unreacted ethyl 2-bromooctanoate. The ethyl-2-methansulphonyloctanoate was separated from the impurities by silica-gel chromatography using 20% ethyl acetate/hexane as the eluent. The isolated yield of the title compound was 0.88 g (39%). This was characterised, and converted to 2-methylsulphonyloctanoic acid as in Example 6.

EXAMPLE 9

Ethyl-2-methanesulphinylacetate

Commercial ethyl-2-methylthioacetate (25.54g, 0.19 mol) was added dropwise to a stirred 0°C solution of sodium metaperiodate (47.17g, 0.22 mol) in a 4:1 water/methanol solution (500 ml). The mixture was allowed to warm up to room temperature and stirred overnight (17h). The inorganic materials which precipitated during the reaction were filtered and water/methanol solvent removed by evaporation to leave an amber coloured oil. The residue was dissolved in dichloromethane (100ml) and washed with brine solution (100ml). The organic layer was separated, dried over magnesium sulphate and concentrated under reduced pressure to give the title compound as an orange oil (19.9g, 70%). An analytically pure sample of the product was obtained by distillation (Bp 150°C at 1 mbar).

Proton n.m.r. showed δ:1.32 (3H, t, $CH_3CH_2O$); 2.78 (3H, s,$CH_3SO$); 3.72 (2H, S, $CH_2$); 4.26 (2H, q, $CH_3CH_2O$).

Ethyl-2-methylthiooct-4-enoate

Hex-1-ene (2.9g, 0.0354 mol) in trifluroacetic acid (TFA) (5 ml) was added dropwise to a stirred O°C solution of ethyl-2-methanesulphinylacetate, prepared as above, (5.0g, 0.0354 mol) and trifluoroacetic anhydride (7.4g, 0.03546 mol) in TFA (20 ml). The solution changed colour from pink to colourless, it was allowed to warm up to ambient temperature and then stirred overnight (17h). The TFA was then removed under reduced pressure and the residue dissolved in ethyl acetate (100ml), washed with saturated sodium bicarbonate (50ml) and brine (25ml). The organic phase was collected, dried ($MgSO_4$) and evaporated under reduced pressure to leave the title compound, (4.5g, 60%) as a yellow oil.

Proton n.m.r. showed δ:0.88 (3H, t, $CH_3$); 1.30 (3H, t, $CH_3CH_2O$); 1.38 (2H, m,$^7$ $CH_2$); 1.98 (2H, m, $^6CH_2$); 2.13 (3H, s, $CH_3S$); 2.40 (1H, m, $^3CH$); 2.58 (1H, m, $^3CH$); 3.24 (1H, m, $CH_3SCH$); 4.20 (2H, q, $OCH_2$); 5.36 (1H, m, CH=); 5.51 (1H, m, CH=).

2-methylthiooct-4-enoic acid

Ethyl-2-methylthiooct-4-enoate (4.16g, 0.019 mol) in methanol (15 ml) was added dropwise to an aqueous

solution of sodium hydroxide (4.1g in water 15 ml). The suspension was then heated to reflux and kept at that temperature for 4 hours before cooling to ambient temperature and stirring overnight (17h). The mixture was acidified to pH 2 with concentrated hydrochloric acid, diluted with water (60 ml) and extracted with ethyl acetate (2 x 100 ml). The organic phase was collected, dried ($MgSO_4$) and evaporated to give the title compound as a yellow oil (1.59g, 44%).

Proton n.m.r. showed δ:0.99 (3H, m, $CH_3$); 1.38 (2H, bm, $^7CH_2$); 2.00 (2H, m, $^6CH_2$); 2.40 (1H, m, $^3CH$); 2.60 (1H, m, $^3CH$); 3.20 (1H, bt, CHS); 5.40 (1H, m, CH=); 5.55 (1H, M, CH=); 9.10 (1H, bs, OH) .

EXAMPLE 10

Methyl-dec-9-enyl ether (11-Oxadodec-1-ene)

Methyl iodide (46.1g), and dec-9-ene-1-ol (25.0g, 0.16 mol) were added to a stirred suspension of ground potassium hydroxide (35.9g, 0.64 mol) in dimethyl sulphoxide (150 ml). The suspension was cooled to 0°C and then allowed to warm up to ambient temperature with stirring. After 4 hours the mixture consisted of two layers. The mixture was poured into water (100 ml) and extracted with diethyl ether (3 x 50 ml). The combined diethyl ether layers were back extracted with brine (100 ml), dried ($Na_2SO_4$) and concentrated under reduced pressure to give the title compound as a clear, colourless oil (23.3g, 86%).

Proton n.m.r. showed δ:1.22-1.40 (10H, bs, $CH_2)_5$; 1.55 (2H, p, $CH_2CH_2O$); 2.04 (2H, q, $CH_2CH$=); 3.32 (3H, s, $OCH_3$); 3.36 (2H, t, $OCH_2$); 4.95 (2H, m, $CH_2$=); 5.08 (1H, m, CH=).

Ethyl-2-methylthio-13-oxatetradec-4-enoate

Methyldec-9-enyl ether, prepared as above, 3.1, 0.0177 mol) in trifluoroacetic acid (TFA), (3 ml) was added dropwise to a stirred 0°C solution of ethyl-2-methanesulphinyllacetate prepared as in the previous Example, (2.5g, 0.0177 mol) and trifluroacetic anhydride (3.72g, 0.0177 mol) in TFA (12 ml). The clear colourless solution was allowed to warm up to ambient temperature and stirred over the weekend (70h). The TFA was then removed under reduced pressure and the residue dissolved in ethyl acetate (50 ml) and washed with saturated sodium bicarbonate solution (25 ml), (vigourous effervescence) and saturated brine (25 ml). The organic phase was collected, dried over magnesium sulphate and concentrated under reduced pressure to give the crude product as a yellow oil. The product was purified by silica-gel chromatography using a 20% solution of ethyl acetate/hexane as the eluent. Yield of title compound 90%.

Proton n.m.r. showed δ:1.22-1.40 (13H, m, $CH_3)_5$) and $CO_2CH_2CH_3$); 1.58 (2H, p, $^{11}CH_2$); 1.98 (2H, m, $^6CH_2$); 2.14 (3H, fine d, $SCH_3$); 2.38 (1H, m, $^3CH$); 2.58 (1H, m, $^3CH$); 3.18 (1H, t, CHS); 3.32 (3H, s, $OCH_3$); 3.38 (2H, t, $OCH_2$); 4.20 (2H, complex q, $CO_2CH_2$), 5.36 (1H, M, CH=), 5.52 (1H, m, CH=).

2-Methylthio-13-oxatetradec-4-enoic acid

Ethyl-2-methylthio-13-octatetradec-4-enoate, (0. 9g, 0.00298 mol) was added dropwise to a stirred aqueous methanolic sodium hydroxide solution (1.3g NaOH, methanol 5 ml, water 8 ml). The suspension was heated at 80°C for 5 hours and then cooled to ambient temperature and acidified to pH 1 with concentrated sulphuric acid. The mixture was diluted with ethylacetate (60 ml) and washed with water (60 ml), the organic phase was separated, dried over magnesium sulphate and evaporated to leave the title compound (0.43g, 53%) as a pale brown oil.

Proton n.m.r. showed δ:1.22-1.40 (10H, m, $CH_2)_5$); 1.55 (2H, p, $^{11}CH_2$); 1.96-2.12 (2H, m, $^6CH_2$); 2.18 (3H, fine d, $SCH_3$); 2.30-2.44 (1H, m, $^3CH$); 2.54-2.68 (1H, m, $^3CH$); 3.20 (1H, fine t, CHS); 3.34 (3H, s, $OCH_3$); 3.38 (2H, fine t, $OCH_2$); 5.40 (1H, m, CH=); 5.52 (1H, m, CH=).

EXAMPLE 11

Ethyl-2-methanesulphinyl-13-oxatetradec-4-enoate

Ethyl-2-thiomethyl-13-oxatetradec-4-enoate prepared as in the previous example (2.5g, 0.0083 mol) in ethanol (5 ml) was added dropwise to a 0°C solution of sodium metaperiodate (1.9g, 0.0087 mol) in 1:1 methanol/water (50 ml). The resulting white suspension was stirred at ambient temperature for 60 hours. The inorganic materials which had precipitated were filtered and the solvents removed by evaporation. The resulting aqueous oily residue was diluted with dichloromethane (30 ml) and washed with brine (15 ml). The organic phase was then collected, dried ($MgSO_4$) and concentrated in vacuo to furnish the title compound as a light

brown oil (2.08g, 71%) which consists of a mixture of diasterioisomers*.

Proton n.m.r. showed δ:1.22-1.40 (13H, m, $CH_3)_5$ and $CO_2CH_2CH_3$); 1.55 (2H, p, $^{11}CH_2$); 1.98 (2H, m, $^6CH_2$); 2.60*, 2.68* (3H, s, SOCH); 2.70 (2H, bm, $^3CH_2$); 3.32 (3H, s, $OCH_3$); 3.38 (2H, t, $OCH_2$); 3.48*, 3.58* (1H, complex m, CHSO); 4.25 (2H complex q, $CO_2CH_2$); 5.35 (1H, m, CH=); 5.60 (1H, m, CH=). Asterisks denote twin peaks associated with presence of diastereomers.

<u>2-Methanesulphinyl-13-oxatetradec-4-enoic acid</u>

Ethyl-2-methanesulphinyl-13-oxatetradec-4-enoate, as above, (0.4g, 0.0012 mol) in methanol (2 ml) was added to an aqueous solution of sodium hydroxide (0.6 g in water 4 ml). The reaction mixture was heated at reflux temperature (80°C) for 4 hours then cooled to ambient temperature and acidified to pH 2 with concentrated sulphuric acid. A white solid precipitated. The suspension was diluted with water (30 ml) and extracted with ethyl acetate (30 ml). The organic phase was collected, dried ($MgSO_4$) and evaporated under reduced pressure to give the title compound as a buff coloured oil (0.29g, 80%).

Proton n.m.r. showed δ:1.20-1.40 (10H, m, $CH_2)_5$); 1.58 (2H, p, $^{11}CH$); 1.96-2.04 (2H, m, $^6CH_2$); 2.55-1.72 (2H, bm, $^3CH_2$); 2.72*, 2.74* (3H, s, $CH_3SO$); 3.34 (3H, s, $OCH_3$); 3.40, (2H, m, $OCH_2$); 3.68*, 3.70*(1H complex m, CHSO); 5.38 (1H, m, CH=); 5.60 (1H, m, CH=).

Infra red spectrum (liquid film) contained peaks at 3400, 2920, 2850, 1720, 1460, 1240, 1120, 1020 $cm^{-1}$.

<u>EXAMPLE 12</u>

An assessment was made of the ability of several compounds to enhance the extensibility of stratum corneum.

Measurements of extensibility were made by the method described in EP-A-7785.

The stratum corneum samples were from guinea pig foot pads. Measurements were made at a relative humidity of 62% and a temperature of 22°C on batches of six samples of stratum corneum. Measurements were made by using samples treated with a 0.06M aqueous solutions of acids of formula (1) maintained at pH 4.0 with sodium hydroxide. Further measurements were made with 0.12M aqueous solutions.

The compounds tested were those prepared in accordance with Examples 1, 3, and 10 above, 2-hydroxy octanoic acid was also tested as a comparison.

For each compound, at each concentration, an extensibility ratio was calculated as the ratio of the extensibility measurements for treated samples and untreated control samples.

Results are as follows:

| Compound | Extensibility | ratios |
|---|---|---|
| | 0.06M Solution | 0.12M Solution |
| 2-hydroxy octanoic acid (comparison) | 1.30 | 2.79 |
| 2-thiol octanoic acid | 2.95 | 5.94 |
| 2-methylthiooctanoic acid | 5.66 | 8.85 |
| 2-methylthio-13-oxatetradec-4-enoic acid | 2.02 | 2.29 |

<u>EXAMPLE 13</u>

Creams were prepared with the following basic formulations:

| Ingredients | %w/w |
|---|---|
| Butane-1,3-diol | 10.0 |
| Sodium chloride | 2.0 |
| Silicone oil | 20.2 |
| Propyl paraben | 0.10 |
| Methyl paraben | 0.20 |
| Whitener (titanium dioxide) | 0.20 |
| Petroleum jelly | 0.50 |
| Mineral oil | 1.5 |
| Lactic acid | 5.0 |
| Fragrance | 0.15 |
| Sodium hydroxide | to pH 5.5 |
| Water | balance to 100% |

A test cream also contained 0.37% of 2 thiol octanoic acid together with 0.38% of 2-methylthio octanoic acid in addition to the basic formulation.

A comparative cream consisted only of the above basic formulation.

The creams were stored, and repeatedly challenged by daily inoculation with a yeast (Candida parapsilosis). The test procedure was as follows:

i) introduce aseptically 99ml of the cream into a 250ml flask and add lml of inoculum of the yeast to provide a dilution of $16^6$ cells/ml in the composition.

ii) mix and incubate at 28°C for 24 hours.

iii) remove 1ml of incubated composition from the flask.

iv) add 1ml of inoculum as in step (i).

v) mix and continue incubation at 28°C for a further 24 hours.

Steps (iii) to (v) were repeated daily. The 1ml of composition removed each day was tested for the presence of viable yeast cells by diluting to 10ml with aqueous peptone solution, then mixing lml of the resulting diluted solution with molten agar in a petri dish so as to make an agar plate. The plate was incubated for 3 days at 28°C after which the number of yeast colonies on the plate was counted. The concentration of viable yeast cells in the cream under test was then calculated from this. The failure point in this test was taken as 100 yeast cells per ml on two successive days.

The cream containing 2-thiol octanoic acid and 2-methylthiooctanoic acid did not reach the failure point after 46 days whereas the comparative cream reached the failure point, i.e. showed yeast contamination after 34 days, even though this comparative cream contained antifungal agents, namely methyl and propyl paraben and lactic acid.

EXAMPLE 14

This example illustrates an oil-continuous (i.e. water-in-oil) cream containing 2-methylthio octanoic acid (preparation given in Example 1) is

| Ingredients | %w/w |
|---|---|
| Silicone oils | 24.00 |
| Whitener | 0.15 |
| Humectants | 5.00 |
| 2-methylthiooctanoic aicd | 1.00 |
| Lactic acid | 5.00 |
| Potassium hydroxide | 4.00 |
| Water | 60.85 |
| | 100.00 |

The skin cream, having a pH value of 5, is made by adding gradually to a mixture of silicones and whitener an aqueous mixture of the remaining ingredients and homogenising.

EXAMPLE 15

This example illustrates an oil-continuous (i.e. water-in-oil) cream containing 2-thiol octanoic acid (preparation given in Example 3) evening primrose oil and sunscreens.

| Ingredients | %w/w |
|---|---|
| Silicone oils | 25.00 |
| Whitener | 0.15 |
| Evening primrose oil | 3.00 |
| Humectants | 5.00 |
| Sunscreens | 4.00 |
| 2-thiol octanoic acid | 1.50 |
| Sodium hydroxide | 2.00 |
| Sodium chloride | 2.00 |
| Lactic acid | 5.00 |
| Water | 52.35 |
| | 100.00 |

The skin cream, having a pH value of 4.5, is made by adding gradually to a mixture of silicones and whitener an aqueous mixture of the remaining ingredients and homogenising.

EXAMPLE 16

This example illustrates a oil-continuous (i.e. water-in-oil) gel containing 2-methylthio octen-4-enoic acid prepared as in Example 9.

| Ingredients | %w/w |
|---|---|
| Emulsifiers | 20.00 |
| Silicone oils | 20.00 |
| Humectant | 11.00 |
| 2-methylthio oct-4-enoic acid | 1.00 |
| Sodium hydroxide | 4.55 |
| Lactic acid | 5.00 |
| Water | 38.45 |
| | 100.00 |

The gel, having a pH value of 5.5, is made by adding to the silicone oils an aqueous mixture of the remaining ingredients and homogenising.

EXAMPLE 17

This example illustrates a water-continuous (i.e. oil-in-water) cream containing 2-methylthio octanoic acid.

| Ingredients | %w/w |
|---|---|
| Thickener | 0.50 |
| Whitener | 0.15 |
| Humectant | 13.50 |
| Emulsifiers | 10.35 |
| Silicone oil | 7.60 |
| 2-methylthiooctanoic acid | 1.00 |
| Sodium hydroxide | 5.00 |
| Lactic acid | 3.00 |
| Water | 58.09 |
| | 100.00 |

The skin cream, having a pH value of 4, is made by adding the emulsifiers and whitener to a heated mixture of the thickener, humectant and 75% of the water. The remaining ingredients are added as an aqueous mixture with further homogenising.

EXAMPLE 18

This example illustrates a water-continuous (i.e. water-in-oil) cream containing 2-methane sulphinyl-13-oxatetradec-4-enoic acid, evening primrose oil and sunscreens.

| Ingredients | %w/w |
|---|---|
| Thickener | 0.50 |
| Whitener | 0.20 |
| Humectant | 10.00 |
| Evening primrose oil | 2.00 |
| Sunscreens | 3.00 |
| Emulsifiers | 10.50 |
| Silicone oil | 7.60 |
| 2-methane sulphinyl-13-oxatetradec-4-enoic acid | 1.00 |
| Triethanolamine | 6.00 |
| Lactic acid | 4.00 |
| Water | 55.20 |
| | 100.00 |

The skin cream, having a pH value of 6, is made by adding to a heated mixture of emulsifiers, silicone oil and whitener a mixture of the thickener, humectant and 75% of the water and homogenising. The remaining ingredients are added as an aqueous mixture with further homogenising.

EXAMPLE 19

The example illustrates a night cream containing 2-thiol octanoic acid and beeswax.

| Ingredients | %w/w |
|---|---|
| Silicone oil | 21.00 |
| Emulsifiers | 15.25 |
| Beeswax | 8.00 |
| Lanolin | 2.50 |
| 2-thiol octanoic acid | 2.00 |
| Potassium hydroxide | 5.00 |
| Water | 46.25 |
| | 100.00 |

The night cream, haing a pH value of 6.5, is made by adding to a mixture of emulsifiers, silicone oil, beeswax and lanolin to a mixture f the reamining ingredients and homogenising.

EXAMPLE 20

This example illustrates a lotion suitable for application to the hands containing 2-methanesulphinyl octanoic acid (preparation as in example 4) and lanolin.

| Ingredient | %w/w |
|---|---|
| Emulsifiers | 10.00 |
| Lanolin | 2.50 |
| 2-methanesulphinyl octanoic acid | 3.00 |
| Triethanolamine | 4.50 |
| Water | 80.00 |
| | 100.00 |

EXAMPLE 21

This example illustrates a water-continuous lotion containing 2-methanesulphonyl-octanoic acid, prepared as in Example 5.

| Ingredient | %w/w |
|---|---|
| Emulsifiers | 3.00 |
| Silicone oil | 5.00 |
| Thickener | 0.35 |
| Humectant | 9.45 |
| 2-methanesulphonyl octanoic acid | 1.50 |
| Ammonium hydroxide | 3.95 |
| Ammonium chloride | 2.00 |
| Water | 74.75 |
| | 100.00 |

EXAMPLE 22

This example illustrates a cleansing lotion containing 2-methanesulphonyl hexanoic acid (preparation as in Example 6) and beeswax.

| Ingredients | %w/w |
|---|---|
| Mineral oil | 45.00 |
| Emulsifier | 3.20 |
| Beeswax | 8.00 |
| Thickener | 10.00 |
| Perfume | 0.20 |
| 2-methanesulphonyl hexanoic acid | 1.00 |
| Triethanolamine | 4.00 |
| Water | 28.60 |
| | 100.00 |

The cleansing lotion, having a pH of 5.5, is made by adding to a mixture of emulsifier, mineral oil and beeswax a mixture of the remaining ingredient and homogenising.

EXAMPLE 23

The example illustrates a facial-washing foam containing 2-methylthio 13-oxatetradec-4-enoic acid, prepared as in Example 9, and azulene.

| Ingredient | %w/w |
|---|---|
| Emulsifier | 20.00 |
| Thickener | 3.00 |
| Foam Booster | 25.00 |
| Humectant | 10.00 |
| Azulene crystals | 0.25 |
| Bentone | 0.50 |
| 2-methylthio 13-oxatetradec-4-enoic acid | 2.50 |
| Potassium hydroxide | 4.50 |
| Water | 34.25 |
| | 100.00 |

EXAMPLE 24

This example illustrates a conventional soap bar containing 2-thiol octanoic acid.

| Ingredient | %w/w |
|---|---|
| Anionic detergent | 18.00 |
| Foam aid | 8.00 |
| Sodium hydroxide | 12.00 |
| Hardening agent | 2.00 |
| Alkaline silicate | 2.00 |
| Calcite | 12.00 |
| Talc | 10.00 |
| 2-thiol octanoic acid | 2.00 |
| Water | 34.00 |
| | 100.00 |

EXAMPLE 25

This example illustrates an all-purpose face-mask containing 2-methylthiooctanoic acid.

| Ingredient | %w/w |
|---|---|
| Kaolin | 30.00 |
| Mineral oil | 10.00 |
| Paraffin wax | 10.00 |
| Bentonite | 4.00 |
| 2-methylthio octanoic acid | 1.40 |
| Sodium hydroxide | 4.20 |
| Phytoconcentrol camomile | 0.25 |
| Water | 40.15 |
| | 100.00 |

EXAMPLE 26

This example illustrates a solution used to condition hair containing 2-methylthio-13-oxatetradec-4-enoic acid.

| Ingredient | %w/w |
|---|---|
| Emulsifier | 0.80 |
| 2-methylthio-13-oxatetradec-4-enoic acid | 0.50 |
| Sodium chloride | 0.50 |
| Sodium hydroxide | 2.00 |
| Water | 96.20 |
| | 100.00 |

EXAMPLE 27

The example illustrates a nail strengthener suitable for treating dryness and brittleness, containing 2-thiol octanoic acid.

| Ingredient | %w/w |
|---|---|
| Humectant | 10.00 |
| Mineral oil | 10.00 |
| 2-thiol octanoic acid | 2.00 |
| Potassium hydroxide | 4.50 |
| Water | 73.50 |
| | 100.00 |

EXAMPLE 28

The example illustrates a lotion suitable for treatment of nails, containing 2-methylthiooctanoic acid and beeswax.

| Ingredient | %w/w |
|---|---|
| Propant-1,2-diol | 50.00 |
| Ethanol | 10.00 |
| Beeswax | 5.00 |
| 2-methylthiooctanoic acid | 3.00 |
| Sodium chloride | 3.00 |
| Sodium hydroxide | 4.25 |
| Water | 24.75 |
| | 100.00 |

This lotion, having a pH value of 4.3 is made by homogenising a mixture of the ingredients.

EXAMPLE 29

This example illustrates a water-in-silicone oil emulsion which is a sunscreen.

| Ingredient | %w/w |
|---|---|
| Silicone surfactant | 10.00 |
| (Dow Corning DC 3225C) | |
| Volatile siloxane | 14.00 |
| (Dow Corning DC 345) | |
| Mineral oil | 1.50 |
| Ultrafine titanium dioxide | 5.00 |
| (water-dispersible) | |
| 2-thiol octanoic acid | 1.00 |
| Lactic acid | 5.00 |
| Butylene glycol | 10.00 |
| Sodium chloride | 2.00 |
| Sodium hydroxide to yield pH | 5.00 |
| Perfume | qs |
| Water | balance to 100% |

EXAMPLE 30

This example illustrates a water-in silicone oil emulsion which is a sunscreen.

| Ingredient | %w/w |
|---|---|
| Volatile siloxane | 8.2 |
| (Dow Corning 345 Fluid) | |
| Silicone surfactant | 12.0 |
| (Dow Corning 3225C) | |
| Mineral oil | 1.5 |
| Petroleum jelly | 0.5 |
| Parsol MCX (octyl methoxycinnamate) | 1.5 |
| Ultrafine titanium dioxide | 1.0 |
| (oil dispersible) | |
| 2-methylthiooct-4-enoic acid | 1.0 |
| Sodium chloride | 2.0 |
| Butylene glycol | 10.0 |
| Sodium hydroxide to pH | 4.5 |
| Perfume | qs |
| Water | balance to 100% |

## Claims

1. A cosmetically acceptable composition suitable for topical application to human skin, hair or nails which comprises:
(i) from 0.1 to 99.9% by weight of an acid or salt thereof having the general formula

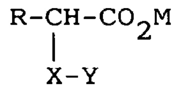

(I)

in which
R is a substituted or unsubstituted alkyl or alkenyl chain of 4 to 28 carbon atoms, optionally interrupted by a heteroatom;
M is hydrogen or a water-solubilising cation;
X is -S-,

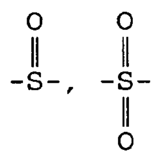 or 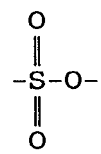

and Y is an alkyl or alkenyl group of up to 4 carbon atoms or, if X is -S-, Y may additionally be hydrogen,
(ii) from 0.1 to 99.9% of a cosmetically acceptable vehicle.

2. A composition according to claim 1 in which R contains 6 to 16 carbon atoms.

3. A composition according to claim 2 in which R contains 6 or 8 carbon atoms.

4. A composition according to claim 1, claim 2 or claim 3 in which X is -S- and Y is hydrogen or alkyl of 1 to 4 carbon atoms.

5. A composition according to any one of the preceding claims in which R is interrupted by an oxygen atom.

6. A composition according to any one of the preceding claims in which R incorporates olefinic unsaturation.

7. A composition accoding to any one or of the preceding claims, in which the compound of formula (I) forms from 0.5 to 10% by weight.

8. A composition according to any of claims 1 to 7 which is an emulsion.

9. A composition according to any of claims 1 to 8 which is a lotion.

10. A composition according to claim 8 which is a cream.

11. A method of treating skin by applying thereto a compound of formula (I) as defined in any of claims 1 to 6.

12. Use of a compound of formula (I) as defined in any of claims 1 to 6 for the preparation of a composition for application to human skin.

European Patent Office

# EUROPEAN SEARCH REPORT

Application Number
EP 93 30 4962

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | FR-A-1 469 512 (J. V. MORELLE ET AL.)<br>* the whole document *<br>--- | 1-4,7-12 | A61K7/04<br>A61K7/06<br>A61K7/48 |
| X | FR-A-1 578 008 (J. MORELLE ET AL.)<br>* the whole document *<br>--- | 1-4,7-12 | |
| A | US-A-2 719 814 (J. W. HAEFELE)<br>* column 2, line 60 - line 65; claims 1-11 *<br>--- | 1 | |
| A | EP-A-0 297 436 (C. R. THORNFELDT)<br>* claims 1-13 *<br>--- | 1 | |
| A | GB-A-2 077 620 (SOC. NAT. ELF)<br>* claims 1-19; example 7 *<br>----- | 1 | |
| | | | TECHNICAL FIELDS SEARCHED (Int. Cl.5 ) |
| | | | A61K |

The present search report has been drawn up for all claims

| Place of search | Date of completion of the search | Examiner |
|---|---|---|
| THE HAGUE | 30 AUGUST 1993 | WILLEKENS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)